(19) Europäisches Patentamt / European Patent Office / Office européen des brevets

(11) **EP 3 174 595 B1**

(12) **EUROPÄISCHE PATENTSCHRIFT**

(45) Veröffentlichungstag und Bekanntmachung des Hinweises auf die Patenterteilung:
**07.06.2023 Patentblatt 2023/23**

(21) Anmeldenummer: **15748013.8**

(22) Anmeldetag: **31.07.2015**

(51) Internationale Patentklassifikation (IPC):
***A61N 1/05*** (2006.01)

(52) Gemeinsame Patentklassifikation (CPC):
**A61N 1/0597**

(86) Internationale Anmeldenummer:
**PCT/EP2015/067698**

(87) Internationale Veröffentlichungsnummer:
**WO 2016/016438 (04.02.2016 Gazette 2016/05)**

(54) **SEGMENTIERTE FLÄCHENELEKTRODE**

SEGMENTED SURFACE ELECTRODE

ÉLECTRODE DE SURFACE SEGMENTÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA**

(30) Priorität: **31.07.2014 EP 14179231**

(43) Veröffentlichungstag der Anmeldung:
**07.06.2017 Patentblatt 2017/23**

(73) Patentinhaber: **Berlin Heals GmbH**
**10719 Berlin (DE)**

(72) Erfinder: **MÜLLER, Johannes**
**10717 Berlin (DE)**

(74) Vertreter: **Liebetanz, Michael**
**Isler & Pedrazzini AG**
**Giesshübelstrasse 45**
**Postfach 1772**
**8027 Zürich (CH)**

(56) Entgegenhaltungen:
WO-A2-2007/070579    US-A1- 2009 209 840
US-A1- 2010 152 864    US-B1- 7 640 065

**Beschreibung**

TECHNISCHES GEBIET

Die vorliegende Erfindung betrifft eine Elektrode zur Behandlung von organischem Gewebe mittels Gleichstrom mit den Merkmalen des Oberbegriffs des Anspruchs 1.

STAND DER TECHNIK

Aus der WO 2006/106132 A1 ist eine Elektrode zur Behandlung von organischem Gewebe mittels Gleichstrom bekannt.

Aus der US 2010/152864 A1 ist ein Implantat für den Einsatz an einem Knochen mit einem elektrischen Stimulationssystem bekannt, bei dem die Steuerung geeignet ist, die Stromdichte am Implantat-Knochen-Übergang zu begrenzen oder zu steuern.

Aus der WO 2007/070579 A2 ist ein Implantat zur Stimulation der Regeneration von beschädigten Rückenmarksnerven bekannt, bei dem ein Gleichstrom ausreichender Stärke in die Nähe der beschädigten Stellen geführt wird, um die Regeneration anzuregen, ohne eine Stromstärke anzulegen, die zu einer Gewebetoxizität führt.

DARSTELLUNG DER ERFINDUNG

Es ist daher eine Aufgabe der vorliegenden Erfindung, die Wirkung der Behandlung zu verbessern, bei gleichzeitiger Erhöhung der Behandlungssicherheit, insbesondere durch Verringerung einer Ablösungswahrscheinlichkeit.

Diese Aufgabe wird erfindungsgemäss durch Elektroden mit den Merkmalen des Patentanspruchs 1 gelöst.

Vorzugsweise ist das mindestens eine Steuerelement rückseitig auf oder seitlich an der mindestens einen Elektrodenfläche angeordnet.

Das Halten der vorbestimmten Stromdichte an der Elektrode kann durch Steuerung/Regelung des Stromes, bzw. der Spannung erreicht werden. Das Halten kann insbesondere über eine Zeitdauer von mehreren Minuten bis zu einer halben Stunde vorgesehen sein. Anschliessend kann für dieselbe Zeitdauer oder eine verkürzte oder verlängerte Zeitdauer ein Gleichstrom mit umgekehrter Polarität vorgesehen sein; damit kann sicher verhindert werden, dass sich durch die Stromeinleitung elektrolytische Rückstände ausbilden.

Durch das Beibehalten einer vorgängig eingestellten Stromdichte kann sichergestellt werden, dass die Stromdichte weder zu hoch, noch zu niedrig wird. Eine zu hohe Stromdichte kann schädlich für das organische Gewebe sein und eine zu niedrige Stromdichte kann eine verminderte therapeutische Wirkung haben.

Vorzugsweise handelt es sich beim organischen Gewebe um ein Herzgewebe, vorzugsweise einen Herzmuskel. Die erfindungsbemässe Elektrode wird direkt am Herzmuskel, dem sogenannten Epikard angeordnet, so dass eine leitende Verbindung zwischen dem Herzmuskel und der Elektrode resultiert.

Das mindestens eine Steuerelement weist ein erstes Steuerelement auf, welches in einer bevorzugten Ausführungsform als Konstantstromquelle ausgebildet ist, und ein zweites Steuerelement, welches in einer bevorzugten Ausführungsform die Spannung und die Polarität an den Konstantstromquellen festlegt.

Ein erstes Steuerelement ist jeder einzelnen Elektrodenfläche zugeordnet. Dies erlaubt eine präzise Steuerung/Regelung jeder einzelnen Elektrode, was zu einem genaueren Einhalten des vorgegebenen Wertes führt.

Ein zweites Steuerelement ist allen Elektrodenflächen gemeinsam zugeordnet. Das erste Steuerelement ist durch elektrische Leitungen mit dem zweiten Steuerelement verbunden, wobei das zweite Steuerelement zu den Elektrodenflächen beabstandet angeordnet ist. Vorzugsweise ist das zweite Steuerelement jedoch elektrodennah angeordnet, beispielsweise in einem Anschlussbereich der Elektrode mit einer Versorgungsleitung, welche die elektrischen Leitungen zur Steuerung/Regelung der Stromdichte und optional weitere elektrische Leitungen, wie beispielsweise eine Messleitung, mit der z.B. ein EKG oder eine Impedanz gemessen werden kann, oder eine weitere Steuerungs-/Regelungs-Leitung mit einschliesst.

Das zweite Steuerelement ist dem ersten vorgelagert angeordnet und übernimmt übergeordnete Funktionen. Beispielsweise erfolgt die Strom- bzw. Spannungsvorgabe beim zweiten Steuerelement und das erste Steuerelement operiert mit diesem vorgegebenen Betriebsstrom oder dieser vorgegebenen Betriebsspannung. Diese Struktur des ersten und zweiten Steuerelements erlaubt eine kompakte Bauweise der einzelnen Steuerelemente und vereinfacht die Steuerung/Regelung der jeweiligen Stromdichte an der jeweiligen Elektrode.

In einer vorteilhaften Ausführungsform sind mindestens zwei Elektrodenflächen in einem gemeinsamen Elektrodenträger eingelassen. Der Elektrodenträger ist einstückig zusammenhängend ausgebildet. Durch diese Anordnung der Elektrodenflächen lässt sich ein Elektroden-Element realisieren, welches einfach in der Handhabung ist und eine ausreichende Flexibilität aufweist, so dass die Elektrode den Bewegungen des organischen Gewebes folgen kann und ein Ablösen der Elektrode von der Oberfläche des organischen Gewebes verringert oder vermieden werden kann.

In einer weiteren vorteilhaften Ausführungsform ist jede der mindestens zwei Elektrodenflächen mit dem entsprechenden mindestens einen Steuerelement in einem separaten Elektrodenträger eingelassen, wobei diese einzelnen Elektrodenträger durch einen Verbindungsstrang miteinander verbunden sind. Eine derart ausgebildete Elektrode zeichnet sich durch eine hohe Flexibilität aus. Diese Flexibilität stellt sicher, dass sich die Elektrode nicht auf Grund der Bewegungen des organischen Gewebes von dessen Oberfläche ablöst.

Weitere Ausführungsformen mit gleichmässig verteilten, gleichgrossen Elektrodenflächen sind ebenfalls möglich, beispielsweise eine kreis- oder ellipsenförmige Elektrode mit Elektrodenflächen, welche kreis- oder ellipsensegmentförmig sind oder eine ringförmige Elektrode mit ringsegmentförmigen Elektrodenflächen.

Die Anzahl der Elektrodenflächen kann zwei bis zweihundert, vorzugsweise zehn bis einhundert betragen. Es gilt: je grösser die Anzahl der Elektrodenflächen, d.h. je kleiner die Segmentierung, desto unwahrscheinlicher ist eine Ablösung der Elektrode. Ebenfalls ist es so, dass bei einer kleinen Segmentierung der Einfluss der Ablösung einer einzelnen Elektrodenfläche weniger ins Gewicht fällt, was sich vorteilhaft auf die Behandlung auswirkt.

Die Elektrode kann in eine gerade oder ungerade Anzahl von Elektrodenflächen segmentiert sein. Alternativ können mehrere Reihen von Elektrodenflächen seitlich der Längsmittelachse angeordnet sein. Kreis-, ring- oder ellipsenförmige Elektroden können ebenfalls mehrere Reihen von konzentrisch angeordneten Elektrodensegmenten aufweisen.

Ausführungsformen mit ungleichmässig verteilten und/oder ungleichgrossen Elektrodenflächen sind ebenfalls möglich. Bereiche der Elektrode mit kleineren Elektrodenflächen erlauben eine grössere Krümmung. Es können Elektroden derart ausgestaltet werden, dass die Aufteilung und Anordnung der Elektrodenflächen auf die an der Herzoberfläche vorherrschenden Krümmungen angepasst sind. Beispielsweise weist eine Elektrode entlang ihrer Längsmittelachse kleinere Elektrodenflächen auf, welche seitlich von grösseren Elektrodenflächen umgeben sind. Alternativ kann eine Elektrode in ihrem zentralen Bereich grössere und in ihrem Randbereich kleinere Elektrodenflächen aufweisen.

Die Elektrodenflächen können aus dünnen, leitenden biokompatiblen Metallfolien mit niedriger Polarisation, beispielsweise aus Platin oder Platiniridium gefertigt sein. Alternativ können die Elektrodenflächen ein Gewirk oder Gewebe aus dünnen, leitenden biokompatiblen Metalldrähten mit niedriger Polarisation, beispielsweise aus Platin oder Platiniridium gefertigt sein.

Die Gewirk- oder Gewebestruktur hat den Vorteil, dass sie sich sehr gut dem aufliegenden Gewebe anpassen kann. Ebenfalls weisen solche Strukturen eine gewisse Durchlässigkeit für Flüssigkeiten auf, was ein Ansammeln von Flüssigkeiten zwischen dem aufliegenden Gewebe und der Elektrodenfläche zumindest teilweise verhindert.

Die Gewirk- oder Gewebestruktur begünstigt eine zumindest teilweise Verwachsung mit dem aufliegenden Gewebe, beispielsweise mit Bindegewebe, was den Kontakt zwischen dem aufliegenden Gewebe und der Elektrode verbessert.

Die Gewirke oder Gewebe können jeweils in einem einzelnen Elektrodenträger oder in einem gemeinsamen Elektrodenträger eingebettet sein.

Alternativ kann das Gewirk oder Gewebe abwechslungsweise leitende und nicht-leitende Bereich aufweisen, sodass in einem zusammenhängenden Gewirk oder Gewebe einzelne Elektrodenflächen entstehen, welche unabhängig voneinander sind.

Die einzelnen Gewirk- oder Gewebeflächen können von einer elektrisch leitenden Umrahmung eingefasst sein. Eine solche Umrahmung verhindert, dass das Gewirk oder Gewebe durch Bewegung auseinanderfällt. Durch die Abmessungen und Materialeigenschaften der Umrahmung lässt sich die Steifigkeit derselben beeinflussen. Alternativ kann die Umrahmung auch aus einem nicht-leitenden Material gefertigt sein.

Bei dem erfindungsgemässen Verfahren zur Steuerung der Stromdichte (J) an einer erfindungsgemässen Elektrode wird die an jeder Elektrode angelegte Spannung derart gesteuert/geregelt, dass eine für jede einzelne Elektrodenfläche vorbestimmte Stromdichte gehalten wird.

Vorzugsweise wird die Steuerung/Regelung durch ein MOSFET-Element oder ein Bipolar-Transistor realisiert. Diese elektrischen Bauteile zeichnen sich durch eine kompakte Bauweise aus und gewährleisten eine zuverlässige und exakte Steuerung/Regelung.

In einer weiteren Ausführungsform weist die Elektrode mindestens ein Einwegventil auf, welches den Abtransport von Flüssigkeit ermöglicht, welche sich unter der Elektrode ansammelt. Durch das Abführen der Flüssigkeit wird vermieden, dass sich die Elektrode allmählich vom organischen Gewebe ablöst oder das Ablösen erleichtert wird. Ein anhaltend guter Kontakt zwischen der Elektrode und der Oberfläche des organischen Gewebes ist somit sichergestellt.

Bevorzugt ist das mindestens eine Einwegventil innerhalb einer Elektrodenfläche angeordnet. Dadurch wird erreicht, dass die Flüssigkeit dort abgeführt wird, wo sie den grössten negativen Einfluss auf den Kontakt zwischen der Elektrodenfläche und der Oberfläche des organischen Gewebes hat.

In einem anderen bevorzugten Ausführungsbeispiel ist das mindestens eine Einwegventil zwischen benachbarten Elektrodenflächen angeordnet. Die Anordnung zwischen benachbarten Elektrodenflächen erlaubt die Anordnung des mindestens einen Einwegventils im Elektrodenträger und ermöglicht eine Öffnungsfreie Ausgestaltung der Elektrodenflächen.

Vorzugsweise ist das mindestens eine Einwegventil ein Membranventil, mit einer Ventilmembran.

Ein Verfahren zur Steuerung der Stromdichte (J) an einer Elektrode regelt den durch jede Elektrode fliessende Strom (I) derart, dass eine für jede einzelne Elektrodenfläche innerhalb eines vorbestimmten Intervalls vorgesehene Stromdichte (J) gehalten wird. Alternativ wird die Stromdichte (J) für jede der mindestens einen Elektrodenfläche um einen vorbestimmten Wert gehalten.

Durch die Wahl eines Stromdichteintervalls sind in diesem Intervall keine Anpassungen der Voreinstellung der Stromdichte nötig.

Wird die Stromdichte um einen vorbestimmten Wert geregelt, so lässt sich eine behandlungsspezifische Stromdichte einstellen, welche besonders vorteilhaft ist.

Jede erfindungsgemässe Elektrode kann als stromeinspeisende oder als stromempfangende Elektrode verwendet werden.

Sofern die Steuerelektronik nicht auf der oder den Elektroden besteht, wären eine Reihe von Leitungen zwischen der Leistungsversorgung und den Elektroden notwendig; dann sind dies mindestens eine Leitung pro Elektrode, was bei vielen Elektroden zu einem unflexiblen Kabelbaum führt. Hier schafft die Erfindung Abhilfe, da es sich bei der Verbindung zur Steuer- und Energieversorgungseinheit um eine Zweidrahtverbindung, welche ausschliesslich der Stromversorgung dient, handeln kann, während die Steuerung bzw. Regelung durch die in der Elektrode angeordneten Steuerelemente erfolgt.

Weitere Ausführungsformen sind in den abhängigen Ansprüchen angegeben.

## KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:

Fig. 1     eine Einzelelektrode (nicht gemäss der Erfindung);
Fig. 2     eine Ausführungsform einer erfindungsgemässen Elektrode;
Fig. 3     eine weitere Ausführungsform einer erfindungsgemässen Elektrode;
Fig. 4     eine alternative Ausführungsform der Elektrode der Figur 1 (nicht gemäss der Erfindung);
Fig. 5     eine alternative Ausführungsform der Elektrode der Figur 2;
Fig. 6     eine alternative Ausführungsform der Elektrode der Figur 3;
Fig. 7     eine Prinzipskizze der Ansteuerungsschaltung;
Fig. 8     ein MOSFET-Steuerelement;
Fig. 9     ein Bipolar-Steuerelement;
Fig. 10    eine erfindungsgemässe Elektrode im Einsatz; und
Fig. 11    eine erfindungsgemässe Elektrode mit einem Einwegventil.

## BESCHREIBUNG BEVORZUGTER AUSFÜHRUNGSFORMEN

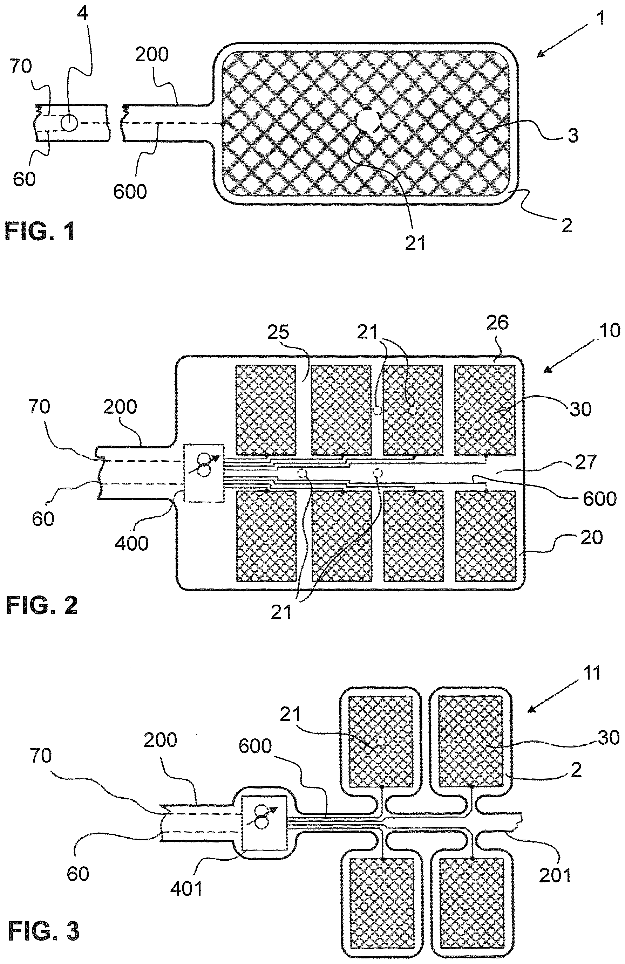

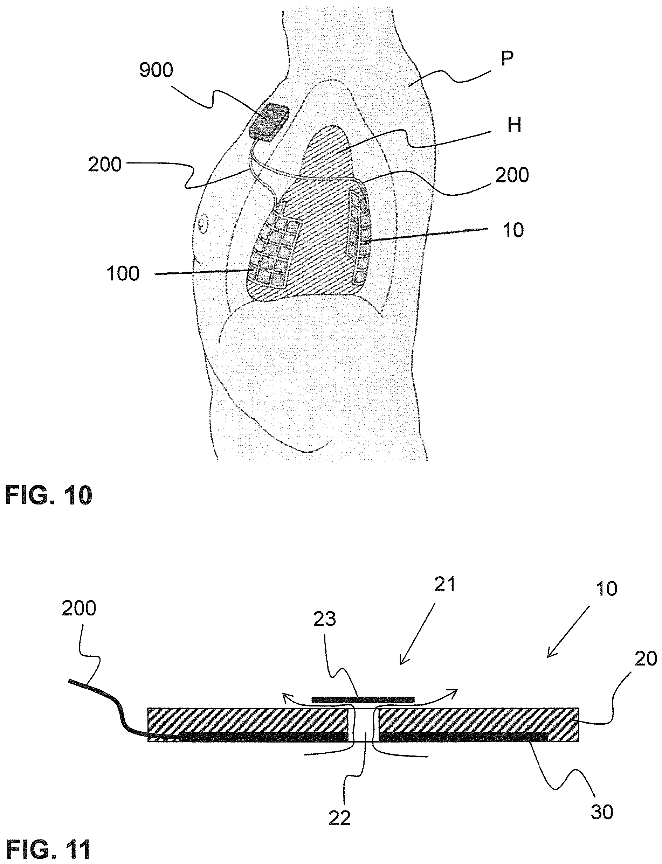

Die Figur 1 zeigt zur Veranschaulichung eine Einzelelektrode 1, nicht gemäss der Erfindung, mit einem ersten Elektrodenträger 2, in welchen eine erste Elektrodenfläche 3 eingelassen ist. Der erste Elektrodenträger 2 umgibt die erste Elektrodenfläche seitlich und rückseitig zumindest teilweise, vorzugsweise vollständig. Die Elektrodenfläche 3 ist durch eine elektrische Leitung 600 mit einem ersten Steuerelement 4 verbunden, welches beabstandet zur Elektrodenfläche 3 in einer Versorgungsleitung 200 angeordnet ist und welches durch elektrische Leitungen 60, 70 mit einem in der Figur 10 dargestellten Steuer und Energieversorgungseinheit 900 verbunden ist. Das erste Steuerelement 4 kann nahe an der Elektrodenfläche 3, d.h. im Verbindungsbereich mit der Versorgungleitung 200, in der Steuer- und Energieversorgungseinheit 900 oder dazwischen angeordnet sein. Durch das erste Steuerelement ist die Stromdichte J der Elektrodenfläche 3 steuer-/regelbar.

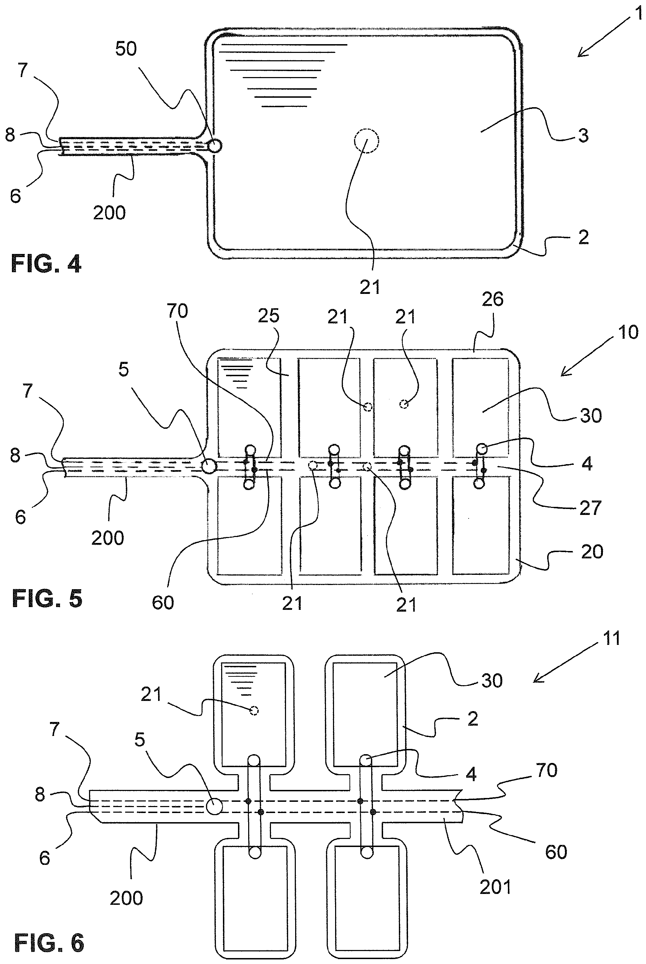

Ein nicht dargestelltes zweites Steuerelement 5 (siehe Figur 5) kann zusammen mit dem ersten Steuerelement 4, benachbart zu diesem oder in der Steuer- und Energieversorgungseinheit 900 angeordnet sein.

Bei einer Elektrodenfläche kann es vorkommen, dass sich diese vom Gewebe ablöst, auf welchem sie vorzugsweise vollständig anliegt. Wird die Elektrode nun so geregelt, dass ein konstanter Strom I durch sie fliesst, so erhöht sich beim Ablösen der Elektrode die Stromdichte, da sich die Fläche reduziert, durch welche der Strom I fliessen kann.

Dabei handelt es sich z.B. bei einer Elektrodenfläche 3 vom 100 Quadratzentimeter und einem Gleichstrom I von 1 Milliampere um eine Stromdichte von 0,01 Milliampere pro Quadratzentimeter. Löst sich die Elektrodenfläche vom Gewebe, so sind beispielsweise nur noch 10 Quadratzentimeter (d.h. ein Zehntel) in Kontakt in welchem Strom fliessen kann. Bei einer Konstantstromregelung würde sich die Stromdichte auf 0,1 Milliampere pro Quadratzentimeter verzehnfachen, da die Fläche durch das Ablösen zehn Mal kleiner geworden ist. Solch hohe Stromdichten sind unerwünscht, da sie beispielsweise bei einem Herz Rhythmusstörungen auslösen können.

Die Elektrode 1 weist optional mindestens ein Einwegventil 21 auf, welches im Wesentlichen eine Öffnung 22 und eine die Öffnung 22 auf der Aussenseite abdeckende Membran 23 aufweist. Eine schematische Schnittansicht des Einwegventils 21 ist in der Figur 11 dargestellt. Beispielsweise ist die Membran aus Silikon. Das mindestens eine Einwegventil 21 ist innerhalb der Elektrodenfläche 3 angeordnet.

Die Figur 2 zeigt eine Ausführungsform einer erfindungsgemässen segmentierten Elektrode 10. Die Elektrode 10 weist mehrere voneinander getrennte Elektrodenflächen 30 auf, welche in einem gemeinsamen, zweiten Elektrodenträger 20 eingelassen sind.

Alle Elektrodenflächen 30 sind gleich gross und sind jeweils paarweise symmetrisch bezüglich der Längsmittelachse der Elektrode angeordnet. Dargestellt sind acht Elektrodenflächen 30, wobei vier auf der einen Seite der Längsmittelachse und vier auf der gegenüberliegenden Seite der Längsmittelachse angeordnet sind.

Der Elektrodenträger 20 weist eine Umrandung 26 auf, welche alle Elektrodenflächen seitlich umschliesst und weist Stege 25 auf, welche die einzelnen Elektrodenflächen voneinander trennt, wobei der Steg, welcher auf der Längsmittelachse liegt ein Mittelsteg 27 ist, welcher die elektrischen Leitungen 600 beinhaltet, welche ein Steuermodul 400 mit den einzelnen Elektrodenflächen 30 verbinden. Das Steuermodul 400 weist mehrere Steuerelemente 4 auf, welche die Stromdichte J in der jeweiligen Elektrodenfläche steuert/regelt. Die Anzahl der Steuerelemente 4 pro Steuermodul 400 korreliert mit der Anzahl der Elektrodenflächen 30.

Elektrische Leitungen 60, 70 verbinden das Steuermodul 400 mit der nicht dargestellten Steuer- und Energieversorgungseinheit 900. Das zweite Steuerelement 5 (nicht dargestellt) kann beim Steuermodul 400, benachbart zu diesem oder bei der Steuer- und Energieversorgungseinheit 900 angeordnet sein. Mit anderen Worten, zwischen der Steuer- und Energieversorgungseinheit 900 und dem Steuermodul 400 handelt es sich insbesondere um elektrische Leitungen 60, 70, die zur Energieversorgung dienen und die Verbindung zur dort vorgesehenen Batterie herstellen können.

In der dargestellten Anordnung liegen sich jeweils zwei Elektrodenflächen 30 bezüglich der Längsmittelachse der Elektrode 10 gegenüber und bilden ein Paar von Elektrodenflächen. Es sind vier solcher Paare nebeneinander in Richtung der Längsmittelachse angeordnet.

Die Verwendung einer Vielzahl von Elektrodenflächen 30 hat den Vorteil, dass wenn sich eine der Elektrodenflächen 30 ablöst, die Stromdichte J der entsprechenden Elektrodenfläche so geregelt werden kann, dass sie einen festgelegten Wert nicht überschreitet und dass die restlichen Elektrodenflächen 30 der segmentierten Elektrode 10 davon unbeeinträchtigt sind.

Die Elektrode 10 weist mindestens ein Einwegventil 21 auf. Das mindestens eine Einwegventil 21 kann innerhalb der Stege 25 angeordnet sein. Es kann alternativ oder zusätzlich innerhalb einer Elektrodenfläche 30 angeordnet sen. Es kann alternativ oder zusätzlich innerhalb des Mittelstegs 27 angeordnet sein. Alternativ oder zusätzlich kann es auch in der Mitte von vier aneinander angrenzenden Elektrodenflächen 30 angeordnet sein, d.h. an Kreuzungen von den Stegen 25 und dem Mittelsteg 27. Das mindestens eine Einwegventil 21 ist derart ausgebildet, dass Flüssigkeit, welche sich unter der Elektrodenfläche 30 ansammelt, nur in eine Richtung durch die Elektrode 10 auf deren Rückseite gelangen kann.

Die Figur 3 zeigt eine alternative Ausführungsform einer erfindungsgemässen segmentierten Elektrode 11. Im Unterschied zur vorherigen Ausführungsform sind die einzelnen Elektrodenflächen 30 jeweils in separaten Elektrodenträger 2 eingelassen, welche durch einen Verbindungsstrang 201 miteinander verbunden sind.

Wie bei der vorangehenden Ausführungsform ist ein Steuermodul 400 vorgesehen, mit welchem die Stromdichte J jeder Elektrodenfläche 30 steuer-/regelbar ist. Wiederum ist das Steuermodul 400 mit elektrischen Leitungen 60, 70 mit der nicht dargestellten Steuerund Energieversorgungseinheit verbunden, welche in der Versorgungsleitung 200 geführt sind.

Vom Steuermodul 400 führen elektrische Leitungen 600 zur jeweiligen Elektrodenfläche 30. Die elektrischen Leitungen 600 sind im Verbindungsstrang 201 geführt. Der Verbindungsstrang weist entlang seiner Längsrichtung seitliche Abzweigungen auf, von welchen jede zu einer Elektrodenfläche 30 führt.

Die Elektrode 10 kann mindestens ein Einwegventil 21 aufweisen, welches innerhalb einer Elektrodenfläche 30 angeordnet ist.

Bei den in den Figuren 1 bis 3 dargestellten Elektroden 1, 10, 11 handelt es sich bei den Elektrodenflächen 3 um ein Gewirke oder Gewebe aus dünnen, leitenden biokompatiblen Metalldrähten mit niedriger Polarisation, beispielsweise aus Platin oder Platiniridium. Alternativ können anstelle der Gewirke oder Gewebe dünne Metallfolien verwendet werden.

Die Figur 4 zeigt eine alternative Ausführungsform der Elektrode der Figur 1, ebenfalls nicht gemäss der Erfindung. Im Unterschied zur Ausführungsform der Figur 1 ist das erste Steuerelement 4 direkt auf der Elektrodenfläche 3 angeordnet. Elektrische Leitungen 6, 7 sind in der Versorgungsleitung 200 geführt und sind mit der nicht dargestellten Steuer- und Energieversorgungseinheit 900 verbunden. Das zweite Steuerelement 5 kann wieder beim ersten Steuerelement 4, benachbart zu diesem oder in der Steuer- und Energieversorgungseinheit 900 angeordnet sein.

Weitere elektrische Leitungen 8 können ebenfalls bis zum zweiten Elektrodenträger 20 führen. Sie können Messleitungen sein, welche zur Messung des EKGs oder der Impedanz benötigt werden.

Die Figur 5, bzw. 6 zeigt eine alternative Ausführungsform der Elektrode der Figur 2 bzw. 3. Im Unterschied zur Ausführungsform der Figur 2 bzw. 3 sind die ersten Steuerelement 4 direkt auf den Elektrodenflächen 30 angeordnet. Die ersten Steuerelemente 4 der Elektrodenflächen 30 sind durch elektrische Leitungen 60, 70 miteinander und mit dem zweiten Steuerelement 5 verbunden.

Bei den in den Figuren 4 bis 6 dargestellten Elektroden 1, 10, 11 handelt es sich bei den Elektrodenflächen 3 um dünne, leitende biokompatiblen Metallfolien mit niedriger Polarisation, beispielsweise aus Platin oder Platiniridium. Alternativ können anstelle der Metallfolien Gewirke oder Gewebe aus dünnen Metalldrähten verwendet werden.

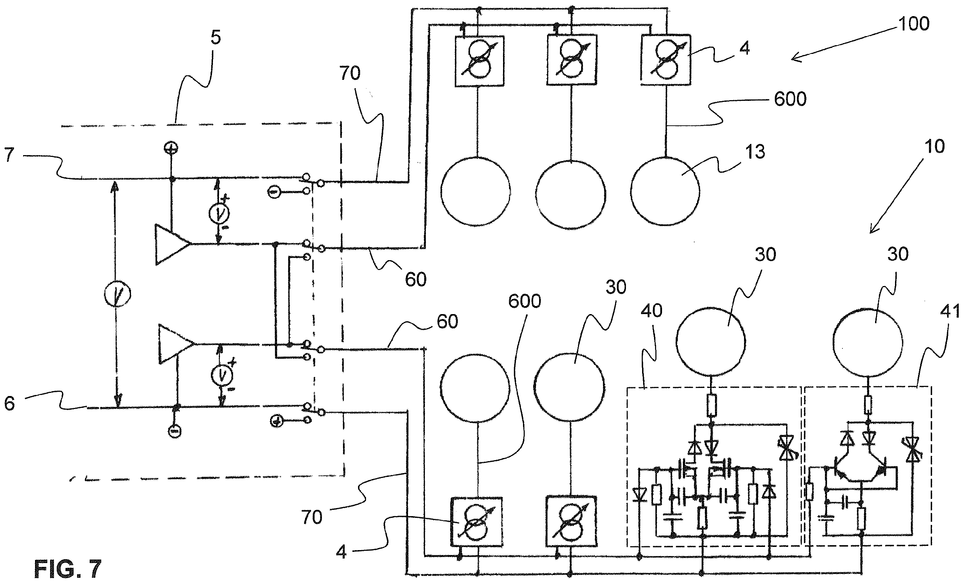

Die Figur 7 zeigt eine Prinzipskizze der Ansteuerungsschaltung einer erfindungsgemässen Elektrodenanordnung mit einer Elektrode 10 und einer Gegenelektrode 100, wie sie in der Figur 10 im Einsatz ist. Die Ansteuerungsschaltung weist für jede Elektrodenfläche 30 ein erstes Steuerelement 4 in der Form einer Konstantstromquelle auf und weist ein zweites Steuerelement 5 auf, welches die Polarität der ersten Steuerelemente 4 festlegt.

Das erste Steuerelement 4 ist vorzugsweise ein Transistor-basiertes Steuerelement, beispielsweise ein erstes Steuerelement 4 mit einem MOSFET-Transistor 9 oder ein alternatives erstes Steuerelement 4 mit einem Bipolar-Transistor 90.

Es ergibt sich aus der Zusammenschau der Figur 2 und der Figur 7 die Möglichkeit, nur die Ansteuerung mit den ersten Steuerelementen 4 am Implantat, an den Patchelektroden, vorzusehen und die zweite Ansteuerung mit den zweiten Steuerelementen 5 auszulagern, wie es durch die Verbindung mit den Bezugszeichen 60 und 70 angedeutet ist, die in dem Ausführungsbeispiel nach Figur 2 die Verbindung nach aussen herstellen, wo sich die Steuer- und Energieversorgungseinheit 900 befindet, somit abweichend gegenüber dem Ausführungsbeispiel nach Figuren 5 und 6.

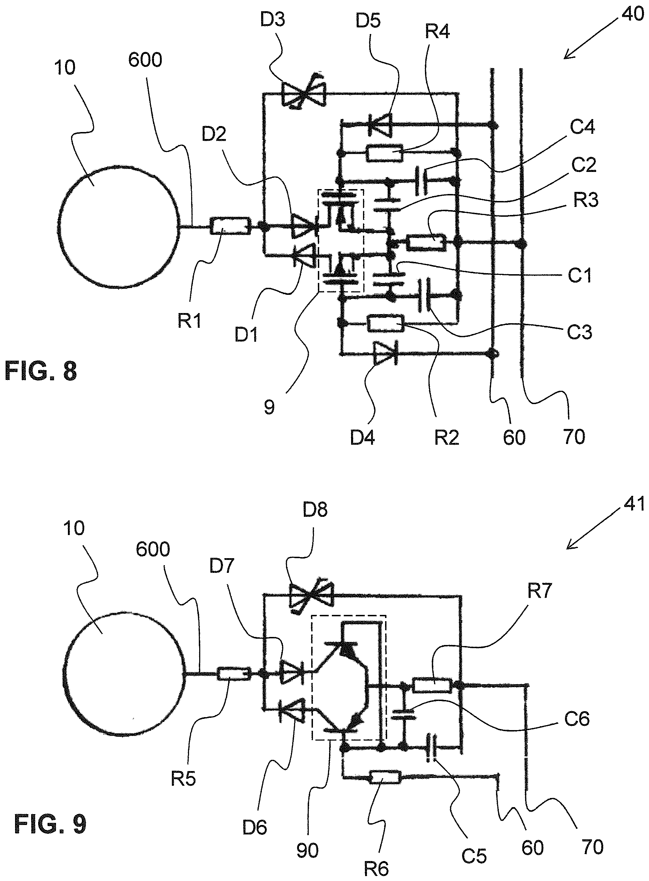

Die Figur 8 zeigt eine Prinzipskizze eines MOSFET-Steuerelements 40 mit einem MOSFET-Transistor 9 und die Figur 9 zeigt eine Prinzipskizze eines Bipolar-Steuerelements 41 mit einem Bipolar-Transistor 90.

Nachfolgend wird das Bipolar-Steuerelement 41 anhand der Figur 9 näher erläutert. Das Prinzip der Stromreglung für eine Stromrichtung (vorgegebene Polarität) kann mit einem Bipolar-Transistor 90, einem Emitterwiderstand R7 und einer Referenzspannung an der Basis des Transistors realisiert werden. Der Emitterwiderstand R7 liegt am Bezugspotential (an der positiven oder negativen Versorgungsspannung für die Stromregler). Der Kollektorausgang wird mit der Elektrode 10, die flächig am Herzmuskel aufliegt, verbunden.

Der Stromkreis wird, wie in der Figur 10 dargestellt, über die zweite auf der gegenüberliegenden Seite des Herzens liegende Gegenelektrode 100 geschlossen. Die gegenüberliegende Elektrode ist ebenfalls mit Stromreglern versehen, um eine definierte Verteilung des Stroms sicherzustellen.

Alternativ kann der Stromkreis über die Steuer- und Energieversorgungseinheit 900 geschlossen werden. In diesem Fall ist jedoch nur eine Elektrode 10 vorhanden.

Dioden D6, D7 in den Kollektorleitungen sorgen für die vorgesehene Stromrichtung, beispielsweise der PNP- und NPN-Transistoren. Unabhängig von der vorgesehenen Stromrichtung erfüllen die Dioden eine Schutzfunktion, um ein Ansteuern der bipolaren Transistoren 90 über die Kollektor-Basis Strecke zu verhindern.

Sicherheitsüberlegungen (Begrenzungen hoher Frequenzen) sind der Grund, auf eine Miller-Kapazität (Gegenkopplungskondensator zwischen Kollektor und Basis) zu verzichten, um Rückwirkungen von der Elektrode über den Kollektor auf die Basis zu verhindern. Die Begrenzung hoher Frequenzen wird durch weitere Kondensatoren erreicht.

Der Spannungsabfall, über den Emitterwiderstand R7 gemessen, bestimmt den Konstantstrom an jeder einzelnen Elektrode 1 bzw. jedem einzelnen Elektrodensegment 10. Diese Spannung ist über die Steuerleitung, die zu den Basen der Transistoren führt, veränderbar. Dadurch kann der Sollstrom (Therapiestrom), der über die entsprechende Elektrodenfläche durch den Herzmuskel fließen soll, eingestellt werden.

Jeder einzelne Transistor regelt sich selbsttätig und hält so bei wechselnden Übergangswiderständen den eingestellten Sollstrom ohne Rückkopplungsschleife zur Steuer- und Energieversorgungseinheit über seinen eigenen Emitter als Spannungsfühler aufrecht.

Der selbsttätige Regelbereich (Innenwiderstandsänderung des Transistors) ist um ein Vielfaches größer als der Einstellbereich über die Steuerspannung.

Mit der Steuerspannung zwischen Hauptleitung und Basis abzüglich der BasisEmitterspannung $U_{BE}$ von ca. 0,6 Volt, die über die Basis-Emitterstrecke abfallen, kann man den Konstantstrom nach dem Ohm'schen Gesetz berechnen:

$$(U - U_{BE}) / R = I$$

mit $U_{BE}$ = 0,6 Volt

Da der angestrebte Strom im pAmpere-Bereich liegt, sollte statt der typischen 0,7 Volt mit 0,6 Volt gerechnet werden.

Der Emitterwiderstand R7 sollte je nach Anzahl der gewünschten Elektrodensegmente und dem verwendeten Strombereich eine Größe von 20-100 Kiloohm haben.

Aus medizinischen Gründen und aufgrund elektrochemischer Effekte soll die Stromrichtung änderbar sein (Wechsel der Polarität). Es wird deshalb ein zweiter Transistor (NPN-PNP) mit einer Diode in der Kollektorleitung benötigt.

Für die jeweils andere Stromrichtung kann in der Steuer- und Energieversorgungseinheit die Polarität der Leitungen zu den Elektroden elektronisch umgeschaltet werden. Dies trifft für die Leitung für die Steuerspannung ebenso zu wie für die Leitungen für den Therapiestrom.

Um den Therapiestrom abzuschalten, kann an mindestens einer der beiden Steuerleitungen die Spannung unter eine Schwelle von 0,4 Volt abgesenkt werden. Aus Sicherheitsgründen ist zusätzlich zu der Absenkung der Spannung unter die erwähnte 0,4 Volt Grenze vorgesehen, die entsprechenden Analogschalter hochohmig zu schalten. Aus diesem Grund können keine Analogschalter mit 3 Anschlüssen verwandt werden.

Der Widerstand R6 erhält eine Relevanz für den Fall, dass das Flächenelement der Elektrode einen hohen Widerstand zu dem Herzmuskel aufweist (Kontaktverlust). In diesem Fall würde der Regeltransistor versuchen, den fehlenden Strom über die Basis zu ziehen. Der Widerstand (R6, ca. 50-150 Kiloohm) begrenzt den Verluststrom auf tolerierbare Werte. Bei einer solchen Konstellation liegt der Widerstand R6 in Reihe mit dem Widerstand R7.

Der sich einstellende Verluststrom kann aus der Steuerspannung abzüglich $U_{BE}$ 0,6 Volt dividiert durch (R6+R7) berechnet werden.

Im Normalbetrieb (guter Kontakt der Elektrode zum Herzmuskel) fließt der größte Teil des Therapiestroms über den Kollektor und nur ein minimaler Reststrom über die Basis. Bei Verstärkungsfaktoren von 100 bis 300 beträgt die Größe des Reststroms nur etwa 1 bis 0,33 Prozent vom Therapiestrom.

Der Widerstand R5 [100-500 Ohm] hat die Funktion eines Schutzwiderstandes und bildet zusammen mit der Diode D8 [zwei gegeneinander geschaltete Zenerdioden 12-14 Volt] einen bidirektionaler Überspannungsschutz D8. Die Spannung für die Zenerdioden ist deshalb so hoch gewählt, weil die Z-Dioden etwa ab 10-12 Volt einen deutlich geringeren Leckstrom aufweisen.

Es geht hier nicht nur um statische Aufladungen, die abgeleitet werden sollen, sondern um einen wirksamen Schutz gegen die Defibrillator-Impulse mit einer Amplitude von bis zu 1500 Volt für 10 Millisekunden (25 Ampere). Dementsprechend muss der Widerstand Impulsspannungen von 1500 Volt aushalten.

Kondensatoren C5, C6 sind zum Schutz gegen einstrahlende Hochfrequenz und gegen die eigene Schwingneigung vorgesehen. Der Regeltransistor kann durch seine stufenlose analoge Regeltätigkeit einen Arbeitspunkt finden, bei dem er ins Schwingen gerät.

Die Kondensatoren sind so dimensioniert, dass die Ausregelzeit nicht die Funktion beeinträchtigt. Ein bewährter Standartwert ist für C6 = 100 Nanofarad und für C5 = 10 Nanofarad, falls die Baugröße der Kondensatoren keine Rolle spielt. Bei der aktuellen Anwendung innerhalb der Elektrode sind auch Werte für C6 von 10 Nanofarad (Spannungsfestigkeit 10 Volt) und für C5 von 5 Nanofarad (Spannungsfestigkeit 20 Volt) akzeptierbar. Die Kapazität darf bei Temperaturänderung nach oben stark abweichen, die angegebene Kapazität darf aber nicht unterschritten werden.

Bei einer vorgegebenen Gesamtfläche F von 6 x 8 Zentimeter ergibt sich eine Fläche F von 48 Quadratzentimeter. Bei einer beispielsweisen Sollstromdichte J von 0,001 Milliampere pro Quadratzentimeter resultiert daraus ein erforderlicher Strom I von 0,048 Milliampere.

Für beispielsweise 12 einzelne Flächen N, wobei jede eine Fläche von 4 Quadratzentimeter hat, bedeutet dies, dass auf jede Fläche ein maximaler Strom I von 0,004 Milliampere entfällt, der maximal als Therapiestrom abgegeben werden darf.

Für R7 ergibt sich hieraus ein Wert R von 40 Kiloohm. Die Steuerspannung berechnet sich wie folgt:

$$R / N = 3{,}33 \text{ Kiloohm x } I = 0{,}16 \text{ Volt} = U,$$

mit R = 40 Kiloohm, N = 12, I = 0,048 Milliampere

$$U + u = 0{,}16 + 0{,}6 = 0{,}76 \text{ Volt} = U_{max}$$

mit u = 0,6 Volt (Spannungsabfall)

Eine maximale Steuerspannung von 0,76 Volt muss für alle 12 Stromregler für eine 12-flächige Multisensorelektrode parallel verfügbar sein.

Die Sollstromdichte kann in einem Intervall von 0,1 bis 20 Mikroampere pro Quadratzentimeter, vorzugsweise zwischen 1 bis 15 Mikroampere pro Quadratzentimeter, bevorzugt zwischen 5 und 10 Mikroampere pro Quadratzentimeter gehalten werden. Alternativ kann die Stromdichte J um einen in den oben genannten Intervallen enthaltenen Wert gehalten werden. Beispielsweise wird die Stromdichte um 10 Mikroampere pro Quadratzentimeter gehalten.

Wird an Stelle eines Bipolar-Transistors 90 ein MOSFET-Transistor 9 verwendet, wie in der Figur 8 dargestellt, erhöht sich die Schwellenspannung in Bezug auf die Steuerspannung auf ca.0,8 Volt (dies ist die Gate-Spannung, bei der MOSFETs gerade anfangen zu leiten), hinzu kommt ein Spannungsabfall über die vor dem Gate liegenden Schottky-Dioden D4, D5, mit 0,25 Volt. Das ergibt auf die Steuerleitung bezogen eine Schwellenspannung von 1,05 Volt.

Das Temperaturverhalten der Dioden wirkt dem Temperaturverhalten der MOSFETs entgegen. MOSFETs 9 werden, im Gegensatz zu bipolaren Transistoren 90, bei Erwärmung hochohmiger.

Die primäre Aufgabe der Dioden D4, D5 ist es, einen Ableitstrom über die internen Schutzdioden der MOSFETs zu verhindern. Es werden nur 2 Dioden für alle Stromregler benötigt. Die Gates der P-Kanal MOSFETs können ebenso direkt parallel geschaltet werden wie alle N-Kanal MOSFETs.

Die Widerstände R2, R4 (je 1 Megaohm) sind hochohmig und sollen für ein zuverlässiges Sperren der MOSFETs sorgen. Wegen der Dioden ist das notwendig. Zwei Widerstände reichen für alle einzelnen Flächen der Multiflächenelektrode aus.

Die Kondensatoren C3, C4 sind ebenso wie die Kondensatoren C1, C2 als Schutz gegen Hochfrequenzeinstreuungen und gegen die Schwingneigung vorgesehen (kein Unterschied zu der Schaltung mit den bipolaren Transistoren). Auch hier reichen zwei Kondensatoren C3, C4 für alle einzelnen Flächen aus. Während C3 und C4 nur einmal für alle Stromregler ausreichend sind, sollten die Kondensatoren C1 und C2 in jeder Stromreglerschaltung vorgesehen werden.

Die Figur 10 zeigt eine erfindungsgemässe Elektrode 10 im Einsatz, wobei sie auf einer ersten äusseren Oberfläche des Herzes H eines Patienten P angeordnet ist und eine Gegenelektrode 100 auf einer zweiten, der ersten im Wesentlichen gegenüberliegenden äusseren Oberfläche des Herzes H angeordnet ist. Vorzugsweise sind die Elektrode 10 im Wesentlichen und die Gegenelektrode 100 auf einer vorderen bzw. einer hinteren Oberfläche des Herzens angeordnet. Alternativ können die Elektroden auch auf einer nach links- bzw. rechts gerichteten Oberfläche des Herzens angeordnet werden. Vorteilhaft an diesen Anordnungen ist, dass der Strom im Wesentlichen durch das Herz bzw. den Herzmuskel fliesst.

Die Gegenelektrode 100 kann identisch oder im Wesentlichen identisch mit der gegenüberliegenden Elektrode 10 ausgebildet sein. Sie kann jedoch auch eine andere Aufteilung bzw. Segmentierung der Elektrodenflächen aufweisen.

Von den Elektroden 10 führen Versorgungsleitungen 200 von jeder Elektrode 10, 100 zu einer gemeinsamen Steuer- und Energieversorgungseinheit 900, welche beispielsweise einer Generator- und Empfangseinheit, eine Telemetrieeinheit und eine Energieversorgungseinheit aufweist.

Eine erfindungsgemässe Elektrode erlaubt die Wirkung der Behandlung zu verbessern, bei gleichzeitiger Erhöhung der Behandlungssicherheit.

## BEZUGSZEICHENLISTE

| | | | |
|---|---|---|---|
| 1 | Einzelelektrode | 40 | MOSFET-Steuerelement |
| 10 | segmentierte Elektrode | 400 | Steuermodul |
| 100 | Gegenelektrode | 41 | Bipolar-Steuerelement |
| 11 | alternative segmentierte Elektrode | 5 | zweites Steuerelement |
| | | 6-8 | elektrische Leitung |
| 2 | erster Elektrodenträger | 9 | MOSFET-Transistor |
| 20 | zweiter Elektrodenträger | 90 | Bipolar-Transistor |
| 200 | Versorgungsleitung | 900 | Steuer- und Energieversorgungseinheit |
| 201 | Verbindungsstrang | | |
| 21 | Einwegventil | R1-R7 | Widerstand |
| 22 | Öffnung | D1-D8 | Diode |
| 23 | Membran | C1-C6 | Kondensator |
| 25 | Steg | I | Strom |
| 26 | Umrandung | J | Stromdichte |
| 27 | Mittelsteg | U | Spannung |
| 3 | erste Elektrodenfläche | P | Patient |
| 30 | zweite Elektrodenfläche | H | Herz |
| 4 | erstes Steuerelement | | |

## Patentansprüche

**1.** Elektrode (10, 11, 100) zur Behandlung von organischem Gewebe mittels Gleichstrom aufweisend einen Elektrodenträger (2, 20), mindestens eine elektrisch leitende Elektrodenfläche (30), welche in den Elektrodenträger (2, 20) eingelassen ist, wobei die mindestens eine Elektrodenfläche (30) mit mindestens einem zugeordneten Steuerelement (4, 50) verbunden ist und wobei das mindestens eine Steuerelement (4, 50) durch mindestens eine elektrische Leitung (6, 7, 8) mit einer Steuer- und Energie-versorgungseinheit (900) verbunden ist, wobei das mindestens eine Steuerelement (4, 50) derart ausgestaltet ist, dass jede einzelne Elektrodenfläche (30) durch das mindestens eine Steuerelement (4, 50) derart ansteuerbar ist, dass eine für jede der mindestens einen Elektrodenfläche (30) innerhalb eines vorbestimmten Intervalls vorgesehene Stromdichte (J) gehalten werden kann oder dass eine Stromdichte (J) für jede der mindestens einen Elektrodenfläche (30) um einen vorbestimmten Wert gehalten werden kann, **dadurch gekennzeichnet, dass** die Elektrode (10, 11, 100) eine segmentierte Elektrode mit mindestens zwei elektrisch leitenden Elektrodenflächen (30) ist, dass das einer Elektrodenfläche (30) zugeordnete Steuerelement jeweils ein erstes Steuerelement (4) aufweist, welche ersten Steuerelemente (4) durch elektrische Leitungen (6, 7) mit einem gemeinsamen zweiten Steuerelement (5) verbunden sind, wobei das zweite Steuerelement (5) zu den Elektrodenflächen (30) beabstandet angeordnet ist.

2. Elektrode (10, 11, 100) gemäss Anspruch 1, wobei jedes erste Steuerelement (4, 50) rückseitig auf oder seitlich an der zugeordneten Elektrodenfläche (30) angeordnet ist.

3. Elektrode (10, 11, 100) gemäss Anspruch 1 oder 2, wobei das zweite Steuerelement (5) in einem Anschlussbereich der Elektrode (10, 11, 100) mit einer Versorgungsleitung (200), welche die elektrischen Leitungen (6, 7) mit einschliesst und weitere elektrischen Leitungen (8) mit einschliessen kann, angeordnet ist.

4. Elektrode (10) gemäss einem der Ansprüche 1 bis 3, wobei die Elektrodenflächen (30) in einen gemeinsamen, zusammenhängenden, einstückig ausgebildeten Elektrodenträger (2, 20) eingelassen sind.

5. Elektrode (10) gemäss einem der Ansprüche 1 bis 3, wobei mindestens zwei der Elektrodenflächen (30) in einen die jeweilige Elektrodenfläche (30) umgebenden Elektrodenträger (2) eingelassen sind und die Elektrodenträger (2) durch einen Verbindungsstrang (200) miteinander verbunden sind.

6. Elektrode (10, 11, 100) gemäss einem der Ansprüche 1 bis 5, wobei das Steuerelement (4) einen MOSFET- oder einen Bipolar-Transistor aufweist.

7. Elektrode (10, 11, 100) gemäss einem der Ansprüche 1 bis 6, wobei die Elektrode (10, 11, 100) mindestens ein Einwegventil (21) aufweist, welches den Abtransport von Flüssigkeit ermöglicht, welche sich unter der Elektrode (10) angesammelt hat.

8. Elektrode (10) gemäss Anspruch 7, wobei das mindestens eine Einwegventil (21) innerhalb einer Elektrodenfläche angeordnet ist.

9. Elektrode (10) gemäss Anspruch 7, wobei das mindestens eine Einwegventil (21) zwischen benachbarten Elektrodenflächen angeordnet ist.

10. Elektrode (10) gemäss einem der Ansprüche 7 bis 9, wobei das mindestens eine Einwegventil (21) ein Membranventil ist.


**Claims**

1. An electrode (10, 11, 100) for the treatment of organic tissue by means of direct current, having an electrode carrier (2, 20), at least one electrically conductive electrode surface (30) which is let into the electrode carrier (2, 20), the at least one electrode surface (30) being connected to at least one associated control element (4, 50) and wherein the at least one control element (4, 50) is connected by at least one electrical line (6, 7, 8) to a control and power supply unit (900), wherein the at least one control element (4, 50) is configured in such a way that each individual electrode surface (30) can be controlled by the at least one control element (4, 50) in such a way that a current density (J) provided for each of the at least one electrode surface (30) can be maintained within a predetermined interval or that a current density (J) for each of the at least one electrode surface (30) can be maintained around a predetermined value, **characterized in that** the electrode (10, 11, 100) is a segmented electrode having at least two electrically conductive electrode surfaces (30), **in that** the control element associated with each electrode surface (30) has a first control element (4), which first control elements (4) are connected by electrical lines (6, 7) to a common second control element (5), the second control element (5) being arranged at a distance from the electrode surfaces (30).

2. The electrode (10, 11, 100) according to claim 1, wherein each first control element (4, 50) is arranged rearwardly on or laterally to the associated electrode surface (30).

3. The electrode (10, 11, 100) according to claim 1 or 2, wherein the second control element (5) is arranged in a connection area of the electrode (10, 11, 100) with a supply line (200) which includes the electrical lines (6, 7) and can include further electrical lines (8).

4. The electrode (10) according to any one of claims 1 to 3, wherein the electrode surfaces (30) are embedded in a common, continuous, integrally formed electrode carrier (2, 20).

5. The electrode (10) according to any one of claims 1 to 3, wherein at least two of the electrode surfaces (30) are embedded in an electrode support (2) surrounding the respective electrode surface (30), and the electrode supports (2) are connected to one another by a connecting strand (200).

6. The electrode (10, 11, 100) according to any one of claims 1 to 5, wherein the control element (4) comprises a MOSFET or a bipolar transistor.

7. The electrode (10, 11, 100) according to any one of claims 1 to 6, wherein the electrode (10, 11, 100) comprises at least one one-way valve (21) allowing the evacuation of liquid accumulated under the electrode (10).

8. The electrode (10) according to claim 7, wherein said at least one one-way valve (21) is arranged within an electrode surface.

9. The electrode (10) according to claim 7, wherein said at least one one-way valve (21) is arranged between adjacent electrode surfaces.

10. The electrode (10) according to any one of claims 7 to 9, wherein the at least one one-way valve (21) is a diaphragm valve.


**Revendications**

1. Electrode (10, 11, 100) pour le traitement de tissus organiques au moyen d'un courant continu, présentant un support d'électrode (2, 20), au moins une surface d'électrode (30) électriquement conductrice, qui est encastrée dans le support d'électrode (2, 20), la au moins une surface d'électrode (30) étant reliée à au moins un élément de commande (4, 50) et l'au moins un élément de commande (4, 50) étant relié par au moins une ligne électrique (6, 7, 8) à une unité de commande et d'alimentation en énergie (900), l'au moins un élément de commande (4, 50) étant conçu de telle sorte que chaque surface d'électrode (30) individuelle puisse être commandée par l'au moins un élément de commande (4, 50) de telle sorte, qu'une densité de courant (J) prévue pour chacune des au moins une surface d'électrode (30) peut être maintenue à l'intérieur d'un intervalle prédéterminé ou qu'une densité de courant (J) pour chacune des au moins une surface d'électrode (30) peut être maintenue autour d'une valeur prédéterminée, **caractérisé en ce que** l'électrode (10, 11, 100) est une électrode segmentée avec au moins deux surfaces d'électrode (30) électriquement conductrices, **en ce que** l'élément de commande associé à une surface d'électrode (30) présente respectivement un premier élément de commande (4), lesquels premiers éléments de commande (4) sont reliés par des lignes électriques (6, 7) à un deuxième élément de commande (5) commun, le deuxième élément de commande (5) étant disposé à distance des surfaces d'électrode (30).

2. Electrode (10, 11, 100) selon la revendication 1, dans laquelle chaque premier élément de commande (4, 50) est disposé à l'arrière sur ou latéralement à la surface d'électrode (30) associée.

3. Electrode (10, 11, 100) selon la revendication 1 ou 2, dans laquelle le deuxième élément de commande (5) est disposé dans une zone de raccordement de l'électrode (10, 11, 100) avec une ligne d'alimentation (200) qui comprend les lignes électriques (6, 7) et peut comprendre d'autres lignes électriques (8).

4. Electrode (10) selon l'une quelconque des revendications 1 à 3, dans laquelle les surfaces d'électrode (30) sont encastrées dans un support d'électrode (2, 20) commun, d'un seul tenant.

**5.** Electrode (10) selon l'une quelconque des revendications 1 à 3, dans laquelle au moins deux des surfaces d'électrode (30) sont encastrées dans un support d'électrode (2) entourant la surface d'électrode (30) correspondante et les supports d'électrode (2) sont reliés entre eux par un cordon de liaison (200).

**6.** Electrode (10, 11, 100) selon l'une quelconque des revendications 1 à 5, dans laquelle l'élément de commande (4) comprend un transistor MOSFET ou un transistor bipolaire.

**7.** Electrode (10, 11, 100) selon l'une quelconque des revendications 1 à 6, dans laquelle l'électrode (10, 11, 100) comporte au moins une valve unidirectionnelle (21) permettant l'évacuation du liquide accumulé sous l'électrode (10).

**8.** Electrode (10) selon la revendication 7, dans laquelle la au moins une valve unidirectionnelle (21) est disposée à l'intérieur d'une surface d'électrode.

**9.** Electrode (10) selon la revendication 7, dans laquelle la au moins une valve unidirectionnelle (21) est disposée entre des surfaces d'électrode adjacentes.

**10.** Electrode (10) selon l'une quelconque des revendications 7 à 9, dans laquelle ladite au moins une valve unidirectionnelle (21) est une valve à membrane.

**FIG. 1**

**FIG. 2**

**FIG. 3**

FIG. 4

FIG. 5

FIG. 6

FIG. 7

**FIG. 8**

**FIG. 9**

EP 3 174 595 B1

**FIG. 10**

**FIG. 11**

16

**IN DER BESCHREIBUNG AUFGEFÜHRTE DOKUMENTE**

**In der Beschreibung aufgeführte Patentdokumente**

- WO 2006106132 A1
- US 2010152864 A1
- WO 2007070579 A2